# EUROPEAN PATENT SPECIFICATION

(11) **EP 1 768 980 B1**
(45) Date of publication and mention of the grant of the patent: **26.11.2008**
(21) Application number: 05778967.9
(22) Date of filing: 19.07.2005
(51) Int. Cl.: C07D 471/04, C07D 471/14

(54) **IMPROVED PROCESS FOR THE MANUFACTURE OF MIRTAZAPINE**
VERBESSERTES VERFAHREN ZUR HERSTELLUNG VON MIRTAZAPIN
PROCEDE AMELIORE DE FABRICATION DE MIRTAZAPINE

(30) Priority: 22.07.2004 ES 200401804
(43) Date of publication of application: 04.04.2007
(73) Proprietor: Medichem, S.A., 08970 Sant Joan Despi, Barcelona (ES)
(72) Inventor: ARNALOT AGUILAR, Carmen, E-08970 Sant Joan Despi,Barcelona (ES)
(74) Representative: Elzaburu Marquez, Alberto
(86) International application number: PCT/EP2005/053493
(87) International publication number: WO 2006/008302

(56) References cited:
- EP-A- 0 129 159
- WO-A-00/62782
- US-A1- 2003 088 094
- US-A1- 2003 135 043

## Description

### Field of the Invention

The present invention relates to a process for preparing mirtazapine.

### Background of the Invention

Mirtazapine, 1,2,3,4,10,14b-hexahydro-2-methyl-pyrazino[2,1,a]pyrido[2,3-c][2]-benzazepine, having the formula (I): 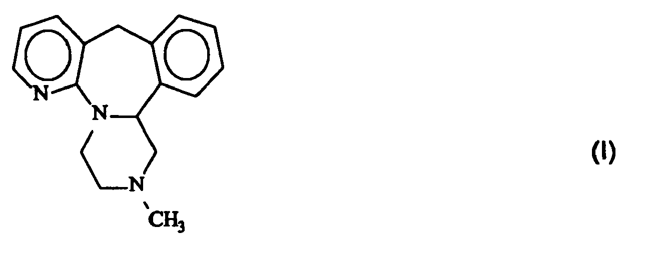 is approved, under the trademark Remeron^{®}, by the U.S. Food and Drug Administration, for the treatment of depression.

Mirtazapine may be made by methods described in U.S. Pat. No. 4,062,848 (Akzona Incorporated). Example 1 of U.S. Pat. No. 4,062,848 explicitly discloses a process for preparing mirtazapine by adding concentrated sulfuric acid to 1-(3-hydroxymethylpyridyl-2)-2-phenyl-4-methylpiperazine of formula (II) at room temperature. 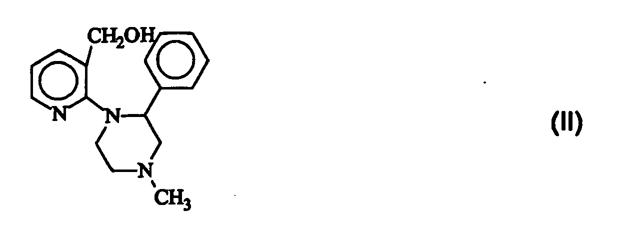 However, there are some defects in this process so that the process is not satisfactory for industrial implementation. Some of the defects are the following:
- Concentrated sulfuric acid is added dropwise at room temperature to a solid compound so the stirring of the mixture is not efficient and the reaction control is difficult.
- The reaction mixture is extracted with chloroform so impurities are also extracted.
- Mirtazapine is crystallized by addition of ether which is very difficult to handle in large scale production.
- Mirtazapine is recrystallized from petroleum ether 40-60 which is very difficult to handle in large scale production.

In Examples 2 and 3 of WO 00/62782 it is disclosed that the same reaction can be carried out by adding 1-(3-hydroxymethylpyridyl-2)-2-phenyl-4-methylpiperazine of formula (II) to concentrated sulfuric acid. In Example 2 the reaction is carried out at room temperature for 4 hours followed by heating for one hour to about 50° to 60°C. In Example 3 the reaction is carried out at 35°C for 6 hours.

In Preparation Example 1 and Preparation Example 2 of EP 1 209 159 A2 it is disclosed that the compound of formula (II) is added to the concentrated sulfuric acid in divided portions at 5° to 30°C and that the mixture is stirred at 30° to 40°C for 8 hours. In addition, mirtazapine is obtained from a mixture of toluene and heptane.

According to the "Detailed Description of the Preferred Embodiments" of patent application EP 1 209 159 A2:
- It is desired that the temperature of the concentrated sulfuric acid (when the pyridinemethanol compound is added to the concentrated sulfuric acid) is 0° to 40°C, preferably 5° to 35°C, in order to suppress heat generation and generation of tarred impurities. (See [0017] on column 3).
- In the case where the pyridinemethanol compound is added to the sulfuric acid, it is preferred that the pyridinemethanol compound is added in divided portions to the concentrated sulfuric acid, in order to cause the reaction to proceed efficiently. For instance, it is preferred that the pyridinemethanol compound is added to the concentrate sulfuric acid in 5 to 20 divided portions. (See [0018] on column 3).

It is important to take into account that the pyridinemethanol compound is added as a solid to the sulfuric acid, which is a disadvantage from an industrial point of view.

The known processes have various drawbacks in that they are complicated, require the observation of particular reaction conditions and do not always lead to mirtazapine of sufficient quality for pharmaceutical use. Accordingly, there is a need for a process useful for the preparation of mirtazapine in high quality on an industrial scale.

### Summary of the Invention

It is an object of the present invention to provide a process for the preparation of mirtazapine which avoids the drawbacks of prior art processes. Further it is an object of the invention to provide a process by which mirtazapine in high purity is obtained.

According to the invention, there is provided a process for preparing mirtazapine comprising the steps of
(a) suspending a compound of the formula (II) 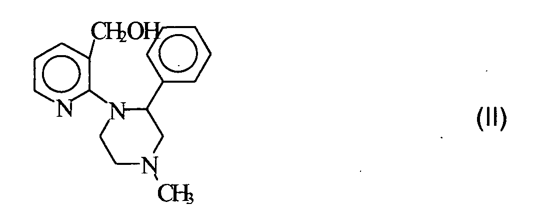 in water to obtain a first mixture;
(b) adding a dehydrating agent to the first mixture to obtain a second mixture; ,
(c) allowing the formation of mirtazapine in the second mixture;
(d) isolating mirtazapine from the second mixture.

One of the preferred embodiments of the invention relates to a process for preparing mirtazapine which comprises purifying of mirtazapine by crystallization from an organic ester solvent.

### Detailed Description of the Invention

The present invention relates to an improved process of making anhydrous mirtazapine from compound of formula (II).

The present invention also provides a new method for making pure anhydrous mirtazapine by purifying crude anhydrous mirtazapine by recrystallization from ethyl acetate.

In the process of the present invention, the compound of formula (II) is suspended in water. A dehydrating agent is added to the resulting mixture and the reaction is carried out at a selected temperature. The completion of the reaction may be monitored by HPLC (High performance liquid chromatography) or thin layer chromatography.

The time needed for the completion of the ring closure varies with the temperature of the reaction. Higher reaction temperatures generally require shorter reaction times, while lower reaction temperatures generally require longer reaction times.

The amount of water is 0.25 to 5 parts by weight, more preferably 0.4 to 1.1 parts by weight based on 1 part by weight of the compound of formula (II).

Dehydrating agents that may be added to the reaction mixture include acids and acid derivatives, such as sulfuric acid, concentrated sulfuric acid, phosphoric acid, phosphorous oxychloride. The dehydrating agents that are particularly preferred are sulfuric acid and concentrated sulfuric acid. The concentration of the concentrated sulfuric acid is preferably in the range of 96 to 99 wt%. The preferable amount of the dehydrating agent is 1 to 6 parts by weight, more preferably 2.5 to 5.0 parts by weight based on 1 part by weight of the compound of formula (II).

It is particularly preferred to use concentrated sulfuric acid as a dehydrating agent. The particularly preferred amount of water is then 0.4 to 0.6 parts by weight of the compound of the formula (II), and the particularly preferred amount of dehydrating agent is 4 to 5 parts by weight of the compound of the formula (II).

The dehydrating agent is preferably added as a thin stream to a mixture of compound (II) and water at a rate that keeps the temperature of the reaction mixture below its reflux temperature. After the addition of the dehydrating agent, it is preferable that the mixture is stirred at a temperature of about room temperature to reflux temperature for about 1 to 24 hours. A temperature of below 90°C, in particular below 80°C, and a reaction time of 1 to 3 hours, in particular about 2 hours, are preferred. These conditions are especially preferred if concentrated sulfuric acid is used as a dehydrating agent.

When the reaction has taken place, the dehydrating agent is diluted or destroyed, for instance, by the addition of a thin stream of the reaction mixture to water or to an aqueous alkali solution. It is preferred that the temperature of the reaction mixture during the addition is from 0° to 30°C. When the dehydrating agent is diluted or destroyed with water, then the mixture is made alkaline by the addition of an aqueous alkali solution. It is preferred that during basification there is present a non-water miscible solvent in order to keep mirtazapine dissolved all time. Any alkali can be used, including but not limited to, sodium hydroxide, potassium hydroxide, sodium carbonate, sodium bicarbonate and ammonium hydroxide. Among them, sodium hydroxide and ammonium hydroxide are preferred.

In a preferred embodiment of the process, the reaction mixture is added to water and a non-water miscible solvent is added before basification. In another preferred embodiment, the reaction mixture is added to an aqueous alkali solution. In this case, the non-water miscible solvent has previously been added to the aqueous alkali solution or to the reaction mixture. Furthermore, it is possible to add water or an aqueous alkali solution to the reaction mixture. Also in this case it is preferred that a non-water miscible solvent is present during the contact of the reaction mixture and any basic component.

Common non-water miscible solvents such as toluene or dichloromethane can be used. Toluene is preferred. During the extraction of crude mirtazapine, the pH of the aqueous alkaline layer is adjusted, if necessary, so that the pH becomes not less than 8, becomes 8 to 10, preferably 8.5 to 9.5.

The isolated non-water miscible layer containing mirtazapine can be treated with a desiccant to remove moisture therefrom, if desired. The desiccant can be any conventional desiccant, including but not limited to, anhydrous sodium sulfate, anhydrous magnesium sulfate and molecular sieves. Alternatively, azeotropic distillation can be used to remove moisture.

In addition, a decolorizing agent is also preferably added to the non-water miscible layer containing mirtazapine, in order to improve the quality attributes like colour and purity of the resulting anhydrous mirtazapine crystals. The decolorizing agent can be any conventional decolorizing agent, including but not limited to, alumina, activated alumina, silica and charcoal. The decolorization temperature is preferably between room temperature and 80°C, more preferably below 40°C.

Crude anhydrous mirtazapine is obtained by removing the non-water miscible solvent by distillation and adding a different solvent which allows the formation of crystalline anhydrous mirtazapine.

The distillation of the non-water miscible solvent can be carried out by any distillation means, preferably by distillation under reduced pressure. The reduced pressure is such that the temperature during distillation is below 50°C, more preferably below 40°C. Solvent is added to the residue of distillation and the distillation under reduced pressure is continued. Then solvent is added to the residue and the mixture is heated to a suitable temperature. Suitable temperatures include, for example, the reflux temperature of the solvent Crude anhydrous mirtazapine precipitates upon cooling of the reaction mixture, preferably to -10° to 10°C, more preferably to 0° to 5°C. After cooling, the mixture is stirred at 0° to 5°C for at least 1 hour and for up to about 6 hours, more preferably for up to about 4 hours to increase the yield of the anhydrous mirtazapine crystal. The anhydrous crude mirtazapine crystals can preferably be collected by filtration or centrifugation.

The wet crude anhydrous mirtazapine crystals are preferably recrystallized again. The crude anhydrous mirtazapine is mixed with fresh solvent and heated to reflux temperature. The amount of solvent is the necessary amount to obtain a solution at reflux temperature. The solution is cooled to 0° to 5°C and stirred at this temperature for about 6 hours, more preferably for about 4 hours, to increase the yield of the anhydrous mirtazapine crystal. The anhydrous mirtazapine crystals can preferably be collected by filtration or centrifugation. The collected anhydrous mirtazapine crystals are dried, preferably under reduced pressure, to reduce the residual solvent in the anhydrous mirtazapine crystals. The drying temperature is preferably 20° to 70°C, more preferably 40°C to 60°C. More preferably the reduced pressure is about 100 mm Hg, and the product is dried at about 40°C for about 2 hours, followed by about 4 hours at about 60°C to remove the residual solvent from the anhydrous mirtazapine crystals.

The solvent which allows the formation of crystals of anhydrous mirtazapine is an organic ester, preferably an organic acetate, more preferably ethyl acetate.

The starting compound of formula (II) is commercially available. Alternatively, compound (II) can be prepared following the methods described in U.S. Pat. No. 4,062,848 and purified by recrystallization from ethyl acetate, if necessary.

### Example 1

This example shows the preparation of mirtazapine using 1 part compound of formula (II) + 0.5 parts water + 4.6 parts dehydrating agent (parts are in weight) and keeping the reaction mixture for 2 h below 80°C.

In a suitable reactor are loaded:
- 3.5 kg of deionized water
- 7.0 kg of 1-(3-hydroxymethylpyridin-2-yl)-4-methyl-2-phenylpiperazine (i. e. the compound of formula (II)).

The reactor is cooled down to 10°C and with continuous stirring. 32.2 kg of sulfuric acid (96.10 wt%, corresponding to 30.94 kg H₂SO₄) are added maintaining the temperature below 80°C. After the addition, the reaction mixture is maintained at 75-80°C during 2 hours. Then the reactor content is cooled down to room temperature and added to 40 kg of deionized water (previously cooled at not more than 15°C) keeping the temperature below 25°C.

Mirtazapine is then extracted by addition of 57 kg of toluene and 54 kg of 26 % ammonium hydroxide to adjust the pH to 8.9 - 9.3. The phases are separated and the aqueous phase is re-extracted with 13 kg of toluene.

The phases are separated and the aqueous phase is re-extracted with 8 kg of toluene.

The organic phases are loaded into a suitable reactor and washed with 61 kg of deionized water.

The organic extracts are treated with anhydrous sodium sulfate and filtered. Then the solution is 2 times de-coloured with active charcoal and filtered. Toluene is distilled off under vacuum without exceeding 40°C and mirtazapine is crystallized from ethyl acetate and filtered.

The wet solid obtained is re-crystallized from ethyl acetate, filtered and dried at 40°C and then at 60°C at a pressure of not more than about 100 mm of Hg. 4.7 Kg. (Molar yield: 72%) of anhydrous crystalline mirtazapine are obtained.

The solid is then milled, sieved through a 500 µm screen and blended for at least 2 hours.

The HPLC purity is 99.7%. Residual solvents (as determined by gas chromatography): toluene below detection limit of 100 ppm, ethyl acetate 299 ppm.

### Example 2

This example shows the preparation of mirtazapine using 1 part compound of formula (II) + 0.5 parts water + 4.6 parts dehydrating agent (parts are in weight) and keeping the reaction mixture for 2.5 h at 40°C + 1 h at 60°C + 1 h at 80°C.

To a suspension of 20 g of 1-(3-hydroxymethylpyridin-2-yl)-4-methyl-2-phenylpiperazine in 10 ml of deionized water, 50 ml of sulfuric acid (96.10 wt%) is added dropwise at 24 to 34°C during 15 minutes. Then, the solution is heated to 40°C and maintained for 2 hours and 30 minutes, after to 60°C and maintained 1 hour and finally to 80°C and maintained 1 hour. As a result, a solution is obtained.

At room temperature, 50 ml of water are slowly added. Next 188 ml of toluene are added and then the pH of the mixture is adjusted to about 9 with 28% ammonium hydroxide. The temperature during the pH adjustment is maintained below 30°C.

This solution is allowed to separate into two layers and the aqueous layer is extracted again with toluene.

Then the organic phases are joined and washed with 175 ml of water. Thereafter the toluene is dried with sodium sulfate and decolorized with 0.6 g of charcoal. After 45 minutes, the solution is filtered. The solvent is evaporated under reduced pressure to dryness. The HPLC purity is 99.45%.

21 ml of ethyl acetate is added to the residue, heated to reflux temperature and then cooled to 5°C and stirred at the same temperature for one hour and filtered.

The resulting crystals are washed with 2 ml of ethyl acetate to give wet crude anhydrous mirtazapine. The HPLC purity is 99.94%.

The crude mirtazapine is crystallized with 15 ml of ethyl acetate. Thereafter the mixture is cooled to 0-5°C and stirred at the same temperature for 1 hour.

After filtering and washing with 2 ml of ethyl acetate, the wet crystals are dried under reduced pressure at 60°C until constant weight, to give purified anhydrous mirtazapine crystals. The HPLC purity is 99.98%. Residual solvents (as determined by gas chromatography): toluene below detection limit of 100 ppm, ethyl acetate 175 ppm.

### Example 3

This example shows the preparation of mirtazapine using 1 part compound of formula (II) + 0.5 parts water + 4.6 parts dehydrating agent (parts are in weight) and keeping the reaction mixture for 7 h at 60°C.

To a suspension of 20 g of 1-(3-hydroxymethylpyridin-2-yl)-4-methyl-2-phenylpiperazine in 10 ml of deionized water, 50 ml of sulfuric acid (96.10 wt%) is added dropwise at 20 to 60°C during 20 minutes. Then the solution is heated to 60°C and maintained for 7 hours.

As a result, a solution is obtained which is cooled to 0-5°C and 50 ml of water are slowly added. Next 188 ml of toluene are added and then the pH of the mixture is adjusted to about 9 with 28% ammonium hydroxide. The temperature during the pH adjustment is maintained below 25°C.

This solution is allowed to separate into two layers and the aqueous layer is extracted again with toluene.

Then the organic phases are joined and washed with 175 ml of water. Thereafter the toluene is dried with sodium sulphate and decolorized with 0.6 g of charcoal. After 1h and 15 minutes, the solution is filtered. The solvent is evaporated under reduced pressure to dryness to give 17.45 g of solid residue. The HPLC purity is 98.48%.

21 ml of ethyl acetate is added to the residue, heated to reflux temperature and then cooled to 5°C and stirred at the same temperature for one hour and filtered.
The resulting crystals are washed with 2 ml of ethyl acetate to give 16.87 g of wet crude anhydrous mirtazapine. The HPLC purity is 99.20%

The crude mirtazapine is crystallized with 15 ml of ethyl acetate. Thereafter the mixture is cooled to 0-5°C, and stirred at the same temperature for 1 hour.

After filtering and washing with 2 ml of ethyl acetate, the wet crystals are dried under reduced pressure at 70°C until constant weight, to give 15.05 g (molar yield: 80.35%) of purified anhydrous mirtazapine crystals. The HPLC purity is 99.58%. Residual solvents (as determined by gas chromatography): toluene below detection limit of 100 ppm, ethyl acetate 258 ppm.

### Example 4

This example shows the preparation of mirtazapine using 1 part compound of formula (II) + 1 part water + 2.76 parts dehydrating agent (parts are in weight) and keeping the reaction mixture for 1h at a temperature in the range from 60° to 100°C.

To a suspension of 20 g of 1-(3-hydroxymethylpyridin-2-yl)-4-methyl-2-phenylpiperazine in 20 ml of deionized water, 30 ml of sulfuric acid (96.10 wt%) is added dropwise at 20 to 58°C during 15 minutes. Then the solution is gradually heated to 100°C in 1 hour.

As a result, a solution is obtained which is cooled to 22°C and 30 ml of water are slowly added. Next 188 ml of toluene are added and then the pH of the mixture is adjusted to about 9 with 28% ammonium hydroxide. The temperature during the pH adjustment is maintained below 25°C.

This solution is allowed to separate into two layers and the aqueous layer is extracted again with toluene.

Then the organic phases are joined and washed with 175 ml of water. Thereafter the toluene is dried with sodium sulfate and decolorized with 0.6 g of charcoal. After 1h and 15 minutes, the solution is filtered. The solvent is evaporated under reduced pressure to dryness to give 16.39 g of solid residue.

21 ml of ethyl acetate is added to the residue, heated to reflux temperature and then cooled to 5°C and stirred at the same temperature for one hour and filtered.

The resulting crystals are washed with 2 ml of ethyl acetate to give 13.68 g of wet crude anhydrous mirtazapine. The HPLC purity is 97.32%.

The crude is crystallized with 15 ml of ethyl acetate. Thereafter the mixture is cooled to 0-5°C and stirred at the same temperature for 1 hour.

After filtering and washing with 2 ml of ethyl acetate, the wet crystals are dried under reduced pressure at 60°C until constant weight, to give 11.18 g (molar yield: 59.09%) of purified anhydrous mirtazapine. The HPLC purity is 99.40%. Residual solvents (as determined by gas chromatography): toluene below detection limit of 100 ppm, ethyl acetate 321 ppm.

### Example 5

This example shows the preparation of mirtazapine using 1 part compound of formula (II) + 1part water + 2.76 parts dehydrating agent (parts are in weight) and keeping the reaction mixture for 3 h 10 min at 20°C + 1 h 30 min at 75-80°C.

To a suspension of 20 g of 1-(3-hydroxymethylpyridin-2-yl)-4-methyl-2-phenylpiperazine in 20 ml of deionized water, 30 ml of sulfuric acid (96.10 wt%) is added dropwise at 20 to 58°C during 25 minutes. Then, the solution is stirred at room temperature for 3 hours and 10 minutes. Next the solution is heated to 75-80°C for 1 h 30 min.

As a result, a solution is obtained which is cooled to room temperature and 35 ml of water are slowly added. Next 188 ml of toluene are added and then the pH of the mixture is adjusted to about 9 with 28% ammonium hydroxide. The temperature during the pH adjustment is maintained below 25°C.

This solution is allowed to separate into two layers and the aqueous layer is extracted again with toluene.

Then the organic phases are joined and washed with 175 ml of water. Thereafter the toluene is dried with sodium sulfate and decolorized with 0.6 g of charcoal. After 1 h and 15 minutes, the solution is filtered. The solvent is evaporated under reduced pressure to dryness to give 16.65 g of solid residue. The HPLC purity is 98.26%.

21 ml of ethyl acetate is added to the residue, heated to reflux temperature and then cooled to 5°C and stirred at the same temperature for one hour and filtered.

The resulting crystals are washed with 2 ml of ethyl acetate to give 14.49 g of wet crude anhydrous mirtazapine. The HPLC purity is 99.70%.

The crude mirtazapine is crystallized with 18 ml of ethyl acetate. Thereafter the mixture is cooled to 0-5°C and stirred at the same temperature for 1 hour.

After filtering and washing with 2 ml of ethyl acetate, the wet crystals are dried under reduced pressure at 60°C until constant weight, to give 12.1 g (molar yield: 64.60%) of purified anhydrous mirtazapine. The HPLC purity is 99.96%. Residual solvents (as determined by gas chromatography): toluene below detection limit of 100 ppm, ethyl acetate 229 ppm.

### Example 6 (Example of Reference)

This example shows the preparation of mirtazapine using 1 part compound of formula (II) + 4.64 parts dichloromethane + 4.6 parts dehydrating agent (parts are in weight) and keeping the reaction mixture for 16 h at room temperature.

10 g of 1-(3-hydroxymethylpyridin-2-yl)-4-methyl-2-phenylpiperazine are suspended in 35 ml of dichloromethane, then 25 ml of sulfuric acid (98.08 wt%) is added in 10 minutes. The mixture is maintained at room temperature for 16 hours.

Maintaining internal temperature below 25°C, 50 ml of water are added. Next 25 ml of toluene are added and the mixture is cooled. Then the pH of the mixture is adjusted to about 9 with 30% ammonium hydroxide. At this time, 50 ml more of toluene are added and the solution is allowed to separate into two layers and the aqueous layer is extracted again with toluene.

Then the organic phases are joined and the toluene is dried with sodium sulfate and filtered. The solvent is evaporated under reduced pressure to dryness to give 8.76g of a solid residue. The HPLC purity of this residue of mirtazapine is 96.99%.

8 g of the obtained residue are dissolved in 9 ml of hot ethyl acetate and then cooled to 5°C and stirred at the same temperature for one hour and filtered to give 7.05 g of wet crude anhydrous mirtazapine. The HPLC purity is 98.70 %.

The crude mirtazapine is crystallized with 5 ml of ethyl acetate. Thereafter the mixture is cooled to 0-5°C and stirred at the same temperature for 1 hour.

After filtering, the wet crystals are dried under reduced pressure at 60°C until constant weight, to give 6.4 g (molar yield: 74.98%) of purified anhydrous mirtazapine. The HPLC purity is 99.73 %. Residual solvents (as determined by gas chromatography): toluene below detection limit of 100 ppm, dichloromethane below detection limit of 100 ppm, ethyl acetate 154 ppm.

## Claims

1. A process for preparing mirtazapine comprising the steps of
(a) suspending a compound of the formula (II) 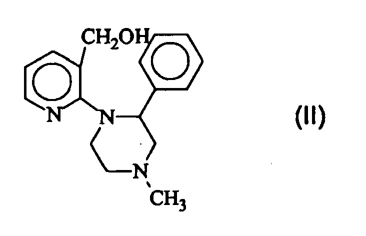 in water to obtain a first mixture;
(b) adding a dehydrating agent to the first mixture to obtain a second mixture;
(c) allowing the formation of mirtazapine in the second mixture;
(d) isolating mirtazapine from the second mixture.

2. The process of claim 1 wherein the dehydrating agent is selected from the group consisting of sulfuric acid, concentrated sulfuric acid, phosphoric acid and phosphorous oxychloride.

3. The process of claim 2 wherein the dehydrating agent is concentrated sulfuric acid.

4. The process of any of claims 1 to 3 wherein the step of allowing the formation of mirtazapine comprises keeping the second mixture at a temperature below 90°C for a time of 1 to 4 hours.

5. The process of any of claims 1 to 4 wherein the step of isolating mirtazapine involves contacting the second mixture with water or with an aqueous solution to obtain a third mixture comprising an aqueous phase.

6. The process of claim 5 wherein the pH of the aqueous phase is adjusted to not less than 8.

7. The process of claim 5 or claim 6 wherein the step of isolating mirtazapine involves the addition of a non-water miscible solvent.

8. The process of claim 7 wherein the non-water miscible solvent is selected from the group consisting of toluene and dichloromethane.

9. The process of claim 7 or claim 8 wherein the step of isolating mirtazapine involves the separation of the non-water miscible phase containing mirtazapine from the aqueous phase.

10. The process of claim 9 wherein the aqueous phase is extracted one or more times with a non-water miscible solvent to obtain an additional portion of non-water miscible phase.

11. The process according to claim 9 or claim 10 wherein the non-water miscible phase is subjected to decolorization.

12. The process of any of claims 7 to 11 wherein the step of isolating mirtazapine involves in addition removing the solvent.

13. The process of any of claims 1 to 12 comprising the additional step of purifying the isolated mirtazapine by crystallizing it one or more times from an organic ester solvent.

14. The process of claim 13 wherein the solvent is ethyl acetate.

15. The process of claim 13 or claim 14 wherein the mirtazapine is obtained in anhydrous crystalline form.

16. The process of any of claims 1 to 15 wherein
(a) the compound of the formula (II) is mixed with water to obtain the first mixture;
(b) sulfuric acid is added as a dehydrating agent to obtain the second mixture;
(c) the formation of mirtazapine in the second mixture is allowed keeping the second mixture at a temperature below 90°C for a time of 2 hours;
(d) mirtazapine is isolated from the second mixture by contacting with water, addition of toluene adjustment of the pH of the aqueous phase to 8 to 10, subsequent separation of the toluene phase, decolorization and then removal of the toluene by evaporation.

17. The process of claim 16 comprising the additional step of purifying the obtained mirtazapine by crystallization from ethyl acetate.

## Patentansprüche

1. Verfahren zur Herstellung von Mirtazapin, umfassend die Schritte:
(a) Suspendieren einer Verbindung der Formel (II) 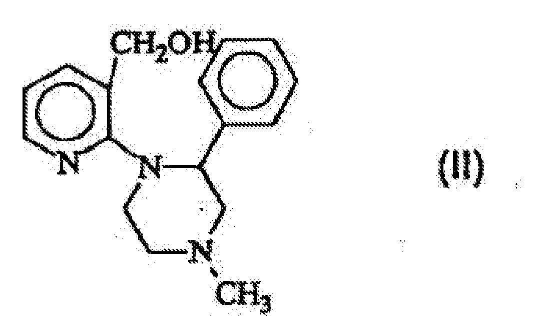 in Wasser, um ein erstes Gemisch zu erhalten;
(b) Hinzufügen eines dehydratisierenden Mittels zum ersten Gemisch, um ein zweites Gemisch zu erhalten;
(c) Zulassen der Bildung von Mirtazapin im zweiten Gemisch;
(d) Isolieren von Mirtazapin aus dem zweiten Gemisch.

2. Verfahren gemäß Anspruch 1, wobei das dehydratisierende Mittel ausgewählt ist aus Schwefelsäure, konzentrierter Schwefelsäure, Phosphorsäure und Phosphoroxychlorid.

3. Verfahren gemäß Anspruch 2, wobei das dehydratisierende Mittel konzentrierte Schwefelsäure ist.

4. Verfahren gemäß einem der Ansprüche 1 bis 3, wobei der Schritt des Zulassens der Bildung von Mirtazapin umfasst, das zweite Gemisch für eine Zeit von 1 bis 4 Stunden bei einer Temperatur unterhalb von 90°C zu halten.

5. Verfahren gemäß einem der Ansprüche 1 bis 4, wobei der Schritt des Isolierens von Mirtazapin das In-Kontakt-Bringen des zweiten Gemischs mit Wasser oder mit einer wässrigen Lösung einschließt, um ein drittes Gemisch zu erhalten, welches eine wässrige Phase umfasst.

6. Verfahren gemäß Anspruch 5, wobei der pH-Wert der wässrigen Phase auf nicht geringer als 8 eingestellt wird.

7. Verfahren gemäß Anspruch 5 oder Anspruch 6, wobei der Schritt des Isolierens von Mirtazapin die Zugabe eines nicht mit Wasser mischbaren Lösungsmittels einschließt.

8. Verfahren gemäß Anspruch 7, wobei das nicht mit Wasser mischbare Lösungsmittel ausgewählt ist aus Toluol und Dichlormethan.

9. Verfahren gemäß Anspruch 7 oder Anspruch 8, wobei der Schritt des Isolierens von Mirtazapin die Trennung der nicht mit Wasser mischbaren Phase, die Mirtazapin enthält, von der wässrigen Phase einschließt.

10. Verfahren gemäß Anspruch 9, wobei die wässrige Phase einmal oder mehrmals mit einem nicht mit Wasser mischbaren Lösungsmittel extrahiert wird, um eine zusätzliche Menge der nicht mit Wasser mischbaren Phase zu erhalten.

11. Verfahren gemäß Anspruch 9 oder Anspruch 10, wobei die nicht mit Wasser mischbare Phase einer Entfärbung unterzogen wird.

12. Verfahren gemäß einem der Ansprüche 7 bis 11, wobei der Schritt des Isolierens von Mirtazapin zusätzlich das Entfernen des Lösungsmittels einschließt.

13. Verfahren gemäß einem der Ansprüche 1 bis 12, umfassend den zusätzlichen Schritt des Reinigens des isolierten Mirtazapins durch einmaliges oder mehrmaliges Kristallisieren aus einem organischen Ester-Lösungsmittel.

14. Verfahren gemäß Anspruch 13, wobei das Lösungsmittel Ethylacetat ist.

15. Verfahren gemäß Anspruch 13 oder Anspruch 14, wobei Mirtazapin in wasserfreier kristalliner Form erhalten wird.

16. Verfahren gemäß einem der Ansprüche 1 bis 15, wobei
(a) die Verbindung der Formel (II) mit Wasser gemischt wird, um das erste Gemisch zu erhalten;
(b) Schwefelsäure als ein dehydratisierendes Mittel hinzugefügt wird, um das zweite Gemisch zu erhalten;
(c) die Bildung von Mirtazapin im zweiten Gemisch zugelassen wird, wobei das zweite Gemisch für eine Zeit von 2 Stunden bei einer Temperatur von unterhalb 90°C gehalten wird;
(d) Mirtazapin aus dem zweiten Gemisch isoliert wird durch In-Kontakt-Bringen mit Wasser, Hinzufügen von Toluol, Einstellung des pH-Wertes der wässrigen Phase auf 8 bis 10, nachfolgende Abtrennung der Toluolphase, Entfärbung und dann Entfernung des Toluols durch Verdampfung.

17. Verfahren gemäß Anspruch 16, umfassend den zusätzlichen Schritt des Reinigens des erhaltenen Mirtazapins durch Kristallisation aus Ethylacetat.

## Revendications

1. Procédé pour la préparation de mirtazapine comprenant les étapes
(a) de mise en suspension d'un composé de la formule (II) 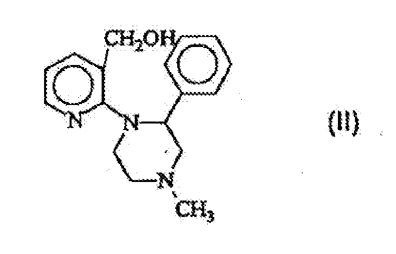 dans l'eau pour obtenir un premier mélange ;
(b) d'addition d'un agent de déshydratation au premier mélange pour obtenir un deuxième mélange ;
(c) de formation de la mirtazapine dans le deuxième mélange ;
(d) d'isolement de la mirtazapine à partir du deuxième mélange.

2. Procédé selon la revendication 1, dans lequel l'agent de déshydratation est sélectionné parmi le groupe constitué de l'acide sulfurique, de l'acide sulfurique concentré, de l'acide phosphorique et de l'oxychlorure phosphoreux.

3. Procédé selon la revendication 2, dans lequel l'agent de déshydratation est l'acide sulfurique concentré.

4. Procédé selon l'une quelconque des revendications 1 à 3, dans lequel l'étape de la formation de mirtazapine comprend le maintien du deuxième mélange à une température inférieure à 90 °C pendant une période de 1 à 4 heures.

5. Procédé selon l'une quelconque des revendications 1 à 4, dans lequel l'étape d'isolement de la mirtazapine met en jeu la mise en contact du deuxième mélange avec de l'eau ou avec une solution aqueuse pour obtenir un troisième mélange comprenant une phase aqueuse.

6. Procédé selon la revendication 5, dans lequel le pH de la phase aqueuse est ajusté à au moins 8.

7. Procédé selon la revendication 5 ou la revendication 6, dans lequel l'étape d'isolement de la mirtazapine met en jeu l'addition d'un solvant non miscible à l'eau.

8. Procédé selon la revendication 7, dans lequel le solvant non miscible à l'eau est sélectionné parmi le groupe constitué du toluène et du dichlorométhane.

9. Procédé selon la revendication 7 ou la revendication 8, dans lequel l'étape d'isolement de la mirtazapine met en jeu la séparation de la phase non miscible à l'eau contenant la mirtazapine de la phase aqueuse.

10. Procédé selon la revendication 9, dans lequel la phase aqueuse est extraite à une ou à plusieurs reprises à l'aide d'un solvant non miscible à l'eau pour obtenir une portion supplémentaire de phase non miscible à l'eau.

11. Procédé selon la revendication 9 ou la revendication 10, dans lequel la phase non miscible à l'eau est sujette à une décoloration.

12. Procédé selon l'une quelconque des revendications 7 à 11, dans lequel l'étape d'isolation de la mirtazapine met en jeu en outre l'élimination du solvant.

13. Procédé selon l'une quelconque des revendications 1 à 12, comprenant l'étape supplémentaire de purification de la mirtazapine isolée par cristallisation à une ou à plusieurs reprises à partir d'un solvant d'ester organique.

14. Procédé selon la revendication 13, dans lequel le solvant est l'acétate d'éthyle.

15. Procédé selon la revendication 13 ou la revendication 14, dans lequel la mirtazapine est obtenue sous une forme cristalline anhydre.

16. Procédé selon l'une quelconque des revendications 1 à 15, dans lequel
(a) le composé de la formule (II) est mélangé à de l'eau pour obtenir le premier mélange ;
(b) l'acide sulfurique est ajouté en tant qu'agent de déshydratation pour obtenir le deuxième mélange ;
(c) la formation de la mirtazapine dans le deuxième mélange est permise en maintenant le deuxième mélange à une température en dessous de 90 °C pendant une période de 2 heures ;
(d) la mirtazapine est isolée du deuxième mélange par mise en contact avec de l'eau, addition de toluène, ajustement du pH de la phase aqueuse à la valeur de 8 à 10, séparation subséquente de la phase de toluène, décoloration et puis élimination du toluène par évaporation.

17. Procédé selon la revendication 16, comprenant l'étape supplémentaire de purification de la mirtazapine obtenue par cristallisation à partir de l'acétate d'éthyle.
